# EUROPÄISCHE PATENTSCHRIFT

(11) **EP 0 271 014 B1**
(45) Veröffentlichungstag und Bekanntmachung des Hinweises auf die Patenterteilung: **10.02.1993**
(21) Anmeldenummer: 87117947.9
(22) Anmeldetag: 04.12.1987
(51) Int. Cl.: C07D 201/04

(54) **Verfahren zur Herstellung von Caprolactam durch Beckmannsche Umlagerung von Cyclohexanonoxim**
Process for preparing caprolactam by the Beckmann rearrangement of cyclohexanone oxime
Procédé de préparation de caprolactame par transposition de Beckmann de cyclohexanonoxime

(30) Priorität: 11.12.1986 DE 3642314
(43) Veröffentlichungstag der Anmeldung: 15.06.1988
(73) Patentinhaber: BASF Aktiengesellschaft, 67063 Ludwigshafen (DE)
(72) Erfinder: De Decker, Emile, D-2710 Hoboken (DE); Oostvogels, Jozef, D-2120 Schoten (DE); van Wauwe, Gerard, B-2520 Edegem (BE); Neubauer, Gerald, Dr., D-6940 Weinheim (DE)

(56) Entgegenhaltungen:
- US-A- 3 914 217
- US-A- 3 953 438
- Chemical Abstracts, Band 80, Nr.15, 15.April 1974, Columbus Ohio USA, K.Konoshita et al., Seite 330; & JP-B-48 39 949 (Toray Industries)

## Beschreibung

Gegenstand der Erfindung ist ein Verfahren zur Herstellung von Caprolactam durch Beckmannsche Umlagerung von Cyclohexanonoxim mit Oleum.

Aus der japanischen Patentveröffentlichung 48-39949 ist ein Verfahren bekannt, bei dem man Cyclohexanonoxim mit Oleum in mehreren hintereinander geschalteten Reaktionszonen umsetzt, wobei man mindestens 70 % des Oleums der ersten Reaktionszone zuführt und Cyclohexanonoxim auf die einzelnen Reaktionszonen verteilt zugibt. Nach einem anderen in der US-PS 3 953 438 beschriebenen Verfahren wird Cyclohexanonoxim mit Oleum in zwei hintereinander geschalteten Umlaufzonen umgesetzt, wobei man den größeren Teil des Cyclohexanonoxims der ersten Reaktionszone und den kleineren Teil des Cyclohexanonoxims der zweiten Reaktionszone zugibt und die gesamte Oleummenge der ersten Reaktionszone zuführt. Entsprechend der US-PS 3 914 217 wird die in der Vorgenannten US-PS beschriebene Arbeitsweise auf drei Umlagerungsstufen ausgedehnt. Mit den Verfahren nach dem Stand der Technik gelingt es zwar die Permanganatabsorptionszahl wesentlich zu erniedrigen. Es hat sich jedoch herausgestellt, daß mit den steigenden Anforderungen an die Qualität des Caprolactams, die so erhaltene Qualität nicht mehr den Anforderungen entspricht.

Es war deshalb die technische Aufgabe gestellt, ein Verfahren zur Herstellung von Caprolactam durch Beckmannsche Umlagerung von Cyclohexanonoxim mit Oleum zur Verfügung zu stellen, bei dem sich das so hergestellte Caprolactam nicht nur durch eine niedere Permanganatabsorptionszahl, sondern auch durch eine niedere UV-Kennzahl sowie einen verminderten Gehalt an Oktahydrophenazin auszeichnet.

Diese technische Aufgabe wird gelöst in einem Verfahren zur Herstellung von Caprolactam durch Beckmannsche Umlagerung von Cyclohexanonoxim mit Oleum bei einer Temperatur von 70 bis 130°C in mindestens einer Umlagerungsstufe, wobei man das aus der Umlagerung erhaltene Reaktionsgemisch anschließend ohne weitere Zugabe von Cyclohexanonoxim in einer Nachverweilzone für einen Zeitraum von 10 bis 600 Minuten auf einer Temperatur von 70 bis 110°C hält.

Das neue Verfahren hat den Vorteil, daß auf einfache Weise die Qualität des erzeugten Caprolactams verbessert wird und insbesondere zusätzlich zur Permanganatabsorptionszahl die Werte für die UV-Kennzahl sowie der Gehalt an Oktahydrophenazin vermindert werden.

In der Regel geht man von schmelzflüssigem Cyclohexanonoxim, z.B. mit einer Temperatur von 80 bis 95°C aus. Das geschmolzene Cyclohexanonoxim hat in der Regel einen Wassergehalt von 0 bis 7 %, vorteilhaft einen Wassergehalt von 3,5 bis 6 Gew.-%. Cyclohexanonoxim wird mit Oleum umgelagert. Vorteilhaft verwendet man Oleum mit einem Gehalt an Schwefeltrioxid von 24 bis 35 Gew.-%. Vorzugsweise setzt man je kg Cyclohexanonoxim 1,1 bis 1,8 kg Oleum ein. Bei der Umlagerung hält man eine Temperatur von 70 bis 130°C, insbesondere 108 bis 118°C, ein.

Die Umsetzung wird beispielsweise in einem Gemisch aus Caprolactam und Schwefelsäure, das im Kreislauf geführt wird, durchgeführt. Über Verteilervorrichtungen werden geschmolzenes Cyclohexanonoxim und getrennt davon über Verteilervorrichtungen Oleum zugeführt. Im Kreislauf hält man ein Gewichtsverhältnis von Schwefelsäure zu Caprolactam von 1,0 bis 2,0 ein und einen Gehalt an freiem Schwefeltrioxid von 1,0 bis 14 Gew.-%. Durch Kühlung wird eine Temperatur von 70 bis 130°C aufrechterhalten, wobei man die 40- bis 150-fache Menge des Kreislaufvolumens im Kreis pumpt. Vor der Zugabestelle für Cyclohexanonoxim und Oleum wird ein Gemisch aus Schwefelsäure und Caprolactam in dem Maße entnommen, wie Cyclohexanonoxim und Oleum zugeführt werden. Die mittlere Verweilzeit im Kreislauf beträgt vorteilhaft 30 bis 120 Minuten.

Nach einer bevorzugten Ausführungsform wird die Umlagerung in mehreren hintereinander geschalteten Stufen, z.B. zwei bis vier hintereinander geschalteten Stufen, durchgeführt. Hierbei gibt man in jeder Stufe Cyclohexanonoxim zu, vorteilhaft in von Stufe zu Stufe abnehmenden Mengen, während mindestens 70 %, insbesondere mindestens 90 %, vorzugsweise die gesamte Menge, der erforderlichen Oleummenge bereits der ersten Stufe zugeführt werden. In einer zweistufigen Umlagerung verfährt man beispielsweise so, daß man in der ersten Stufe 60 bis 95 Gewichtsteile Cyclohexanonoxim sowie das gesamte Oleum zugibt, ein Gewichtsverhältnis von Schwefelsäure zu Caprolactam von 1,0 bis 2,0 und einen Gehalt an freiem Schwefeltrioxid von 2,0 bis 14,0 Gew.% sowie eine Temperatur von 70 bis 130°C aufrechterhält. Das so erhaltene Reaktionsgemisch, das im wesentlichen aus Schwefelsäure, Caprolactam und Schwefeltrioxid besteht, wird in dem Maße, wie Oleum und Cyclohexanonoxim zugeführt werden, in eine zweite Stufe überführt und dort die restlichen 5 bis 40 Gewichtsteile Cyclohexanonoxim zugegeben, wobei ein Gewichtsverhältnis von Schwefelsäure zu Caprolactam im umlaufendem Gemisch von 1,0 bis 1,5 und ein Gehalt an freiem Schwefeltrioxid von 1,0 bis 6 Gew.% aufrechterhalten wird. Im umlaufenden Reaktionsgemisch hält man eine Temperatur von 70 bis 130°C ein.

Erfindungsgemäß wird das aus der Umlagerung erhaltene Reaktionsgemisch vor der Neutralisation in einer Nachverweilzone, z.B. einem Rührkessel, oder insbesondere in einer langgestreckten Nachverweilzone, z.B. einer rohrförmigen Nachverweilzone, für einen Zeitraum von 10 bis 600 Minuten, insbesondere 15 bis 180 Minuten, auf eine Temperatur von 70 bis 110°C, insbesondere 90 bis 100°C, gehalten.

Das so erhaltene Reaktionsgemisch, das im wesentlichen aus Caprolactam, Schwefelsäure, restlichem Schwefeltrioxid und Nebenprodukten besteht, wird mit Ammoniak neutralisiert. Vorteilhaft wird das Reaktionsgemisch in einem Kreislaufsystem einer 35- bis 45 gew.-%igen wäßrigen Ammonsulfatlösung zugeführt, mit dieser vermischt und gasförmiges Ammoniak eingeleitet und bis zu einem pH-Wert von 4 bis 5 neutralisiert. Das sich nunmehr abscheidende Rohlactam wird von der gesättigten Ammonsulfatlösung, z.B. durch Dekantieren, abgetrennt und mit Benzol extrahiert. Nach Abtrennen des Benzols wird das Caprolactam durch Destillation unter vermindertem Druck gereinigt.

Das nach dem erfindungsgemäßen Verfahren erhältliche Caprolactam zeichnet sich durch eine größere Reinheit aus, insbesondere wird die UV-Kennzahl und der Gehalt an Oktahydrophenazin vermindert.

Das Verfahren nach der Erfindung sei an folgenden Beispielen veranschaulicht.

### Vergleichsbeispiel

### Arbeitsweise ohne Nachverweilzone

In einem Kreislaufsystem mit insgesamt 10 m³ Inhalt, bestehend aus Verbindungsleitungen, Pumpe, Kühlern und Entgasungs-/Überlaufgefäß wurde die 70-fache Menge des Kreislaufvolumens im Kreislauf gepumpt. Über eine Verteilervorrichtung wurde die gesamte Cyclohexanonoximmenge als Schmelze von 85°C zugegeben.

Das verwendete Cyclohexanonoxim hatte einen Wassergehalt von 4,2 Gew.-%. Gleichzeitig wurde in einer zweiten Verteilervorrichtung Oleum mit 32 Gew.-% SO₃ zugegeben. Das Verhältnis von Oleummenge zur Oximmenge betrug 1,17 kg/kg. Die Temperatur vor den Verteilervorrichtungen betrug 115°C. Die mittlere Verweilzeit im Kreislaufsystem beträgt etwa 60 Minuten. Die in den Reaktionskreislauf eingebrachten Mengen treten als Reaktionsprodukte am Überlaufgefäß wieder aus dem Kreislaufsystem aus und werden in einem zweiten Kreislaufsystem mit ca. 40 bis 43 %iger wäßriger Ammonsulfatlösung vermischt und mit gasförmigem Ammoniak auf pH 4,6 neutralisiert. Rohlactam scheidet sich aus der fast gesättigten Ammoniumsulfatlösung aus und wird abgetrennt. Das wasserhaltige Rohlactam (ca. 30 % Wassergehalt) wird durch Extraktion mit Benzol vorgereinigt und das so entstandene Extraktlactam wird durch Vakuum-Destillation einer Endreinigung zum Reinlactam unterzogen.

Es werden folgende Kennzahlen im Reinlactam erreicht:

| | |
|---|---|
| Permanganat-Titrationszahl | 2,6 |
| Permanganat-Absorptionszahl | 3,6 |
| UV-Kennzahl | 5,0 |
| Extinktion 290 nm/10 cm Küvette | 0,45 |
| OHP (Oktahydrophenazin) ppm | 0,8 |

### Beispiel

### Arbeitsweise mit Nachverweilzone

Im gleichen Kreislaufsystem wie im Vergleichsbeispiel beschrieben, wird das Cyclohexanonoxim unter den dort angegebenen Bedingungen umgelagert. Das entstandene Reaktionsprodukt wird nun in einem separaten Behälter bei einer Temperatur von 95°C für 120 Minuten verweilen gelassen und anschließend wie beschrieben aufgearbeitet und Reinlactam gewonnen.

Es werden folgende Kennzahlen im Reinlactam erreicht:

| | |
|---|---|
| Permanganat-Titrationszahl | 2,2 |
| Permanganat-Absorptionszahl | 3,1 |
| UV-Kennzahl | 2,5 |
| Extinktion 290 nm/10 cm Küvette | 0,3 |
| OHP (Oktahydrophenazin) ppm | 0,5 |

## Patentansprüche

1. Verfahren zur Herstellung von Caprolactam durch Beckmannsche Umlagerung von Cyclohexanonoxim mit Oleum bei einer Temperatur von 70 bis 130°C in mindestens einer Umlagerungsstufe, dadurch gekennzeichnet, daß man das aus der Umlagerungsstufe erhaltene Reaktionsgemisch anschließend ohne weitere Zugabe von Cyclohexanonoxim in einer Nachverweilzone für einen Zeitraum von 10 bis 600 Minuten auf einer Temperatur von 70 bis 110°C hält.

2. Verfahren nach Anspruch 1, dadurch gekennzeichnet, daß man bei der Umlagerung eine Temperatur von 108 bis 118°C einhält.

3. Verfahren nach den Ansprüchen 1 und 2, dadurch gekennzeichnet, daß man die Umlagerung in zwei oder drei hintereinander geschalteten Umlagerungsstufen durchführt.

4. Verfahren nach den Ansprüchen 1 bis 3, dadurch gekennzeichnet, daß man die gesamte Menge an Oleum und 60 bis 95 Gew.-Teile Cyclohexanonoxim der ersten Umlagerungsstufe und 5 bis 40 Gewichtsteile Cyclohexanonoxim den nachfolgenden Umlagerungsstufen zugibt.

5. Verfahren nach den Ansprüchen 1 bis 4, dadurch gekennzeichnet, daß man in der Nachverweilzone eine Temperatur von 90 bis 100°C einhält.

6. Verfahren nach den Ansprüchen 1 bis 5, dadurch gekennzeichnet, daß man in der Nachverweilzone eine Verweilzeit von 15 bis 180 Minuten einhält.

## Claims

1. A process for preparing caprolactam by Beckmann rearrangement of cyclohexanone oxime with oleum at from 70 to 130°C in at least one rearrangement stage, wherein the reaction mixture obtained in the rearrangement stage is subsequently maintained in a delay zone at from 70 to 110°C for from 10 to 600 minutes without further addition of cyclohexanone oxime.

2. A process as claimed in claim 1, wherein a temperature of from 108 to 118°C is maintained in the rearrangement.

3. A process as claimed in claim 1 or 2, wherein the rearrangement is carried out in two or three rearrangement stages connected in series.

4. A process as claimed in any of claims 1 to 3, wherein the total amount of oleum and from 60 to 95 parts by weight of cyclohexanone oxime are added to the first rearrangement stage and from 5 to 40 parts by weight of cyclohexanone oxime to the subsequent rearrangement stages.

5. A process as claimed in any of claims 1 to 4, wherein a temperature of from 90 to 100°C is maintained in the delay zone.

6. A process as claimed in any of claims 1 to 5, wherein a residence time of from 15 to 180 minutes is maintained in the delay zone.

## Revendications

1. Procédé de préparation de caprolactame par transposition de Beckmann de cyclohexanone-oxime avec de l'oléum à une température de 70 à 130°C dans au moins un étage de transposition, caractérisé en ce que le mélange réactionnel obtenu dans l'étage de transposition est ensuite maintenu à une température de 70 a 110°C, sans autre addition de cyclohexanone-oxime, dans une zone de post-séjour pendant une période de 10 à 600 mn.

2. Procédé selon la revendication 1, caractérisé en ce qu'on maintient une température de 108 à 118°C au cours de la transposition.

3. Procédé selon la revendication 1 ou 2, caractérisé en ce qu'on conduit la transposition dans deux ou trois étages de transposition disposés l'un à la suite de l'autre.

4. Procédé selon l'une quelconque des revendications 1 a 3, caractérise en ce qu'on ajoute la quantité totale d'oléum et 60 à 95 parties en poids de cyclohexanoneoxime dans le premier étage de transposition, et 5 à 40 parties en poids de cyclohexanone-oxime dans les étages de transposition suivants.

5. Procédé selon l'une quelconque des revendications 1 à 4, caractérisé en ce qu'on maintient une température de 90 à 100°C dans la zone de post-séjour.

6. Procédé selon l'une quelconque des revendications 1 à 5, caractérisé en ce qu'on observe un temps de séjour de 15 à 180 mn dans la zone de post-séjour.
